# EUROPÄISCHE PATENTSCHRIFT

(11) **EP 1 946 714 B1**
(45) Veröffentlichungstag und Bekanntmachung des Hinweises auf die Patenterteilung: **28.10.2009**
(21) Anmeldenummer: 07017646.6
(22) Anmeldetag: 10.09.2007
(51) Int. Cl.: A61B 18/14

(54) **Chirurgisches Instrument zur Behandlung von tumorösem Gewebe**
Surgical instrument for treating tumorous tissue
Instrument chirurgical destiné au traitement de tissus tumoraux

(30) Priorität: 16.01.2007 CN 200710000839
(43) Veröffentlichungstag der Anmeldung: 23.07.2008
(73) Patentinhaber: Chang, Chengzhong, Beijing (CN); Sheng, Lin, Beijing (CN)
(72) Erfinder: Chang, Chengzhong, Beijing (CN); Sheng, Lin, Beijing (CN)
(74) Vertreter: Huwer, Andreas

(56) Entgegenhaltungen:
- WO-A-00/36985
- WO-A-2004/093704
- US-A1- 2004 167 517
- US-B1- 6 315 777

## Beschreibung

Die Erfindung betrifft ein chirurgisches Instrument nach dem Oberbegriff von Anspruch 1.

Die bösartigen Tumoren sind einer der Hauptgründe für die Gefährdung des Lebens und der Gesundheit des Menschen. Momentan gibt es noch keine Medikamente, mit denen Tumoren endgültig beseitigt werden können. In der letzten Zeit hat sich die Mikrotechnik gegen Tumor sehr stark entwickelt Insbesondere wegen der geringen Verletzungen, den niedrigen Nebenwirkungen, geringerem Leiden der Patienten und der höheren Abtötungsquote des ursprünglich vorhandenen Tumorgewebes wurde dieser Technik mehr Aufmerksamkeit gewidmet. Zur Zeit gibt es für die Mikro-Technik gegen Tumor hauptsächlich elektrische Geräte, die mit Hochfrequenz, Mikrowellen, Argon-Helium arbeiten sowie die Koagulation mit Ultraschall. Mit Hilfe von Ultraschall, CT oder MRI wird die Energie auf die Stelle des Tumors konzentriert, um das Tumorgewebe zu töten.

Ein aus WO 00/36985 A2 bekanntes chirurgisches Instrument der eingangs genannten Art ist als Mikrowellennadel ausgebildet, die an ihrem proximalen Endbereich einen Halteabschnitt aufweist, an dem ein Hohlschaft angeordnet ist, der den Halteabschnitt etwa in gerader Verlängerung fortsetzt An ihrem distalen Endbereich hat die Mikrowellennadel eine den Hohlschaft fortsetzende HF-Nadelelektrode, die an ihrem freien Ende eine Nadelspitze aufweist In dem Hohlschaft ist eine HF-Leitung angeordnet, die an ihrem einen Ende mit der HF-Nadelelektrode und an ihrem anderen Ende über den Halteabschnitt mit einem Anschluss eines HF-Generators verbunden ist.

Zur Kühlung der HF-Nadelelektrode und des zu behandelnden Gewebes ist in dem Hohlschaft ein Fluidkanal vorgesehen. Dieser hat einen ersten Fluidkanalabschnitt und einen konzentrisch dazu angeordneten zweiten Fluidkanalabschnitt Die Fluidkanalabschnitte sind in Reihe geschaltet und an ihrem distalen Ende miteinander verbunden. An ihrem proximale Ende ist der eine Fluidkanalabschnitt mit einer Eintrittsöffnung und der andere Fluidkanalabschnitt mit einer Austrittsöffnung verbunden, die an der dem Hohlschaft abgewandten Rückseite des Halteabschnitts angeordnet sind. Die ersten und zweiten Fluidkanalabschnitte bilden also eine Flüssigkeitsumlaufbahn. Die Mikrowellennadel bietet zahlreiche Vorteile, nämlich sie ermöglicht ein leichtes Einstechen, geringe Verletzungen, und unabhängig davon, wo sich der Tumor befindet, eine stabile Koagulation.

Bei der Behandlung mit der Mikrowellennadel besteht aufgrund der hohen Temperatur an der Nadelspitze (über 100° C) jedoch die Gefahr, dass das Gewebe verkohlt Die Mikrowellennadel kann deshalb häufig nicht tief genug in das Gewebe eingestochen werden. An dem verkohlten Gewebe können die von der HF-Nadelelektrode ausgesendeten Mikrowellen reflekfiert werden, wodurch im Bereich des Nadelschafts Verletzungen an der Haut und am Gewebe hervorgerufen werden können.

Aus WO 2004/093704 A1 ist ferner ein chirurgisches Instrument zur Behandlung von tumorösem Gewebe bekannt, das einen Hohlschaft hat, der an seinem distalen Endbereich eine HF-Nadelelektrode aufweist und an seinem proximale Endbereich mit mindestens einem Halteabschnitt verbunden ist. Die HF-Nadelelektrode ist über den elektrisch leitenden Hohlschaft an einem HF-Generator angeschlossen. Der Hohlschaft hat ein Außerohr und ein darin befindliches Innenrohr. In dem Hohlschaft ist ein Fluidkanal vorgesehen, der sich im Wesentlichen in Axialrichtung des Hohlschaft erstreckt. Zwischen dem Außenrohr und dem Innenrohr hat der Fluidkanal einen ersten Fluidkanalabschnitt und in dem Innenrohr einen zweiten Fluidkanalabschnitt. Die Fluidkanalabschnitte sind am distalen Endbereich des Hohlschafts miteinander verbunden und weisen an ihrem davon entfernten Ende jeweils einen Fluidanschluss auf. Zur Abgabe von Fluid in das tumoröse Gewebe hat der Hohlschaft an seinem distalen Endbereich eine mit dem Fluidkanal verbundene Austrittsöffnung. Im Bereich der Austrittsöffnung ist auf der Außenmantelflöche des Außerohrs eine die Austrittsöffnung überdeckende Außenhülse angeordnet. Zwischen der Außenmantelfläche und der Innenwand der Außenhülse ist ein Durchlass gebildet, der als Strömungswiderstand dient, über den des Fluid in das zu behandelnde Gewebe gelangen kann. Das chirurgische Instrument hat den Nachteil, dass der Strömungswiderstand nicht verstellt werden kann, wenn der Hohlschaft in das zu behandelnde Gewebe eingeführt ist. Daher kann die Arzt während der Behandlung die Temperatur des behandelten Gewebes nicht oder nur schlecht einstellen.

Es besteht deshalb die Aufgabe, ein chirurgisches Instrument zu schaffen, bei dem die Gefahr der Verkohlung des Gewebes und/oder eines Klebenbleibens der Nadelspitze an dem Gewebe weitgehend vermieden werden und das eine große Koagulationsfläche ermöglicht.

Diese Aufgabe wird mit den Merkmalen des Anspruchs 1 gelöst.

In vorteilhafter Weise ist es dadurch möglich, den Hohlschaft an seinem Außenumfang gleichmäßig und flächig über eine zwischen dem Außen- und Innenrohr fließende Flüssigkeit zu kühlen. Die Flüssigkeit kann eine Kochsalzlösung sein. Während der Behandlung mit den Mikrowellen kann das Gewebe durch die Flüssigkeit befeuchtet werden. Diese sickert in das Gewebe ein und leitet die Wärme ab. Somit wird die Verkohlung und Verklebung des Gewebes vermieden und die Koaguationsfläche wird vergrößert. Dabei ist es sogar möglich, die Behandlung mit einer Chemotherapie zu kombinieren, wenn in der Flüssigkeit ein entsprechendes Medikament enthalten ist. Somit können während der Behandlung eine Verbrennung des Gewebes und/oder Klebenbleibens der Nadelspitze an dem Gewebe weitgehend vermieden werden. Durch die Austrittsöffnung hindurch kann während der Behandlung Flüssigkeit in das Gewebe abgegeben werden, um dieses noch besser zu kühlen und/oder mit mindestens einem in der Flüssigkeit enthaltenen Medikament in Kontakt zu bringen. Durch Verändern des Durchlassquerschnitts der Austrittsöffnung kann der Arzt während der Behandlung die Temperatur am Nadelschaft einstellen und somit die Gefahr einer Gewebeverbrennung wirkungsvoll reduzieren. Die vorzugsweise an dem proximalen Endbereich des chirurgischen Instruments angeordneten Halteabschnitte ermöglichen eine einfache Handhabung und Einstellung des Stellelements entsprechend dem jeweils in das Gewebe abzugebenden Flüssigkeitsvolumenstrom. Das chirurgische Instrument ermöglicht einen einfachen und kostengünstigen Aufbau und ist leicht handhabbar.

Zweckmäßigerweise ist benachbart zu der HF-Nadelelektrode und/oder der mindestens einen Austrittsöffnung zwischen dem ersten Fluidkanalabschnitt und dem zweiten Fluidkanalabschnitt eine Flüssigkeitskammer in dem Hohlschaft vorgesehen ist, die wärmeleitend mit der HF-Nadelelektrode und/oder einem Außenwandbereich des Hohlschafts verbunden ist. Die HF-Nadelelektrode und/oder der Außenwandbereich des Hohlschafts können dann aufgrund der relativ großen Wärmekapazität der in der Flüssigkeitskammer enthaltenen Flüssigkeit noch besser temperiert werden.

Bei einer zweckmäßigen Ausgestaltung der Erfindung ist das Außenrohr drehfest mit der HF-Nadelelektrode verbunden. Es ist aber auch möglich, dass das Innenrohr drehfest mit der HF-Nadelelektrode verbunden und das Außenrohr auf der HF-Nadelelektrode verdrehbar ist. In beiden Fällen kann an dem chirurgischen Instrument eine Skala vorgesehen sein, an welcher die jeweils eingestellte Position des Stellelements bzw. der freie Öffnungsquerschnitt der Auslassöffnung ablesbar ist.

Vorteilhaft ist, wenn der mindestens eine Fluidanschluss seitlich am proximalen Endbereich des chirurgischen Instruments und ein mit der HF-Leitung verbundener HF-Anschluss an dem von der HF-Nadelelektrode beabstandeten Rückseite des chirurgischen Instruments angeordnet sind. Das chirurgische Instrument ist dann noch besser handhabbar.

Das erfindungsgemäße chirurgische Instrument kann folgende Teile aufweisen: ein Gehäuse mit einer Flüssigkeitseinlassöffnung und einem Außenrohrsystem, das ein Außenrohr bildet sowie mit einer Flüssigkeitsauslassöffnung und einem Innenrohrsystem, das ein Innenrohr bildet, ein Anschlussgehäuse für die Mikrowellen, eine Mikrowellenleitung und ein Isolierungshülse sowie die Nadelspitze bilden sich ein Nadelspitzensystem für Mikrowellen. Das Innenrohr- und Außenrohrsystem sind durch das jeweilige Gehäuse verbunden. Am Außenrohrschaft gibt es eine Skala und eine medizinische Spezialisolierungsbeschichtung. Im Vorderteil des Außenrohrs befinden sich die Flüssigkeitsauslassöffnungen. Im Vorderteil des Innenrohres, gegenüber den Flüssigkeitsauslassöffnungen im Außenrohr befindet sich als Stellelement ein Metallverschluss, der geklebt oder eingesteckt ist. Das Innenrohr kann sich gegenüber dem Außenrohr nach links oder rechts sowie nach oben oder nach unten bewegen. Das Nadelspitzensystem ist durch das Anschlussgehäufür die Mikrowellen mit dem Gehäuse des Innenrohrs verbunden. Das Ende der Mikrowellenleitung, welches die Mikrowellen freisetzt, ist durch ein Schweißstück mit der Nadel verbunden. So bildet sich eine Mikrowellennadel. Mit Hilfe der Mikrowellenleitung und dem Schweißstück sowie dem Isolierungsmaterial ist die Nadelspitze mit dem Außenrohr verbunden.

Die Mikrowellenleitung ist im Innenrohr und das Innenrohr befindet sich im Außenrohr. Zwischen dem Innenrohr und Außenrohr bildet sich ein Flüssigkeitskanal. Zwischen dem Innenrohr und der Mikrowellenleitung ist der Flüssigkeitskanal.

Am Außenrohrschaft gibt es eine Skala und eine medizinische Spezialisolierungsbeschichtung. Am Vorderteil befinden sich die Flüssigkeitsauslassöffnungen. Im Vorderteil des Außenrohrs gibt es eine Flüssigkeitskammer, welche den Flüssigkeits-Wärmeumtausch möglich macht.

Im Vorderteil des Außenrohrs befinden sich die Flüssigkeitsauslassöffnungen. Im Vorderteil des Innenrohrs, gegenüber den Flüssigkeitsauslassöffnungen des Außenrohrs gibt es einen Metallverschluss. Das Innenrohr kann sich gegenüber dem Außenrohr nach links oder rechts sowie nach oben oder unten bewegen. Somit können sowohl der Flüssigkeitseinsatz als auch die Flüssigkeitsmenge der Mikrowellennadel gesteuert werden. Der Operateur kann je nach medizinischem Bedarf die Flüssigkeitsauslassöffnungen öffnen oder schließen.

Das Nadelspitzensystem ist durch das Anschlussgehäuse für die Mikrowellen mit dem Gehäuse des Innenrohrs verbunden. Das Ende der Mikrowellenleitung, welches die Mikrowellen freisetzt, ist durch das Schweißstück mit der Nadel verbunden. Somit ist ein Nadelspitzensystem für Mikrowellen gebildet. Das System ist durch die Mikrowellenleitung und das Schweißstück sowie die Isolierung mit dem Vorderteil des Außenrohrs verbunden. Wenn sich das Innenrohrsystem bewegt, bleibt das Nadelspitzensystem stehen.

Das Innenrohrsystem (am Schaft gibt es Flüssigkeitsauslassöffnungen) ist mit Nadelspitzensystem verbunden. Das Außenrohrsystem (am Schaft gibt es Flüssigkeitsauslassöffnungen) kann sich nach links oder rechts sowie nach oben und unten bewegen. So kann die Flüssigkeit eingespritzt werden. Im Innenrohr und Außenrohr entsteht eine Flüssigkeitsumlaufbahn.

Das Außenrohrsystem (am Schaft gibt es Flüssigkeitsauslassöffnungen) ist mit Nadelspitzensystem verbunden. Das Innenrohrsystem (am Schaft gibt es Flüssigkeitsauslassöffnungen) kann sich nach links oder rechts sowie nach oben und unten bewegen. So kann die Flüssigkeit eingespritzt werden. Im Innenrohr und Außenrohr entsteht eine Flüssigkeitsumlaufbahn.

Das Innenrohr und Außenrohr sind ein Rohr. Am Vorderteil des Rohrs gibt es Flüssigkeitsauslassöffnungen, die mit dem Nadelspitzensystem verbunden sind. Sie dient nur zum Flüssigkeitseinspritzen. Es entsteht keine Flüssigkeitsumlaufbahn.

Nachfolgend ist ein Ausführungsbeispiel der Erfindung anhand der Zeichnung näher erläutert. Es zeigt.

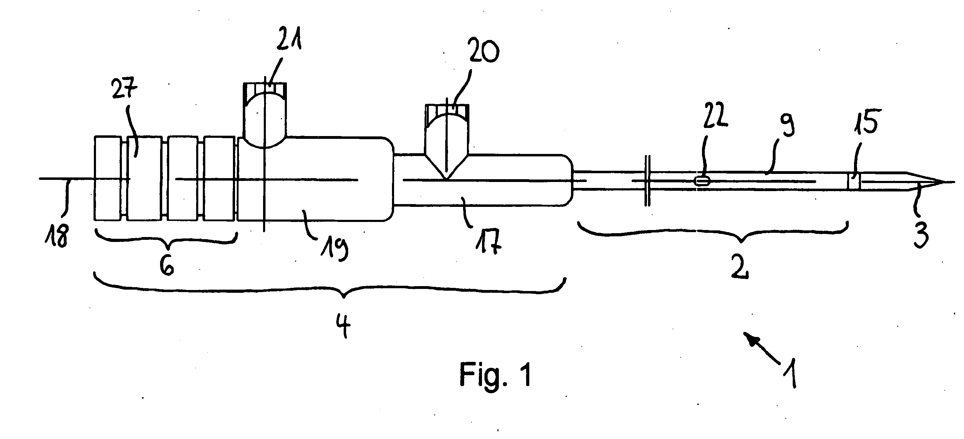

- Fig. 1: eine Seitenansicht eines als Mikrowellennadel mit einem Hohlschaft ausgebildeten chirurgischen Instruments, wobei der Hohlschaft nur teilweise dargestellt ist,
- Fig. 2: einen Längsschnitt durch das chirurgische Instrument, wobei der Hohlschaft nur teilweise dargestellt ist,
- Fig. 3: eine vergrößerte Teilansicht des distalen Endbereichs des chirurgischen Instruments, wobei die HF-Nadelelektrode besonders gut erkennbar ist,
- Fig.4: einen vergrößerten Ausschnitt aus Fig. 2, der eine Austrittsöffnung und einen Flüssigkeitskammer erkennen lässt,
- Fig.5: einen Querschnitt durch den Hohlschaft entlang der in Fig. 4 mit V bezeichneten Ebene, und

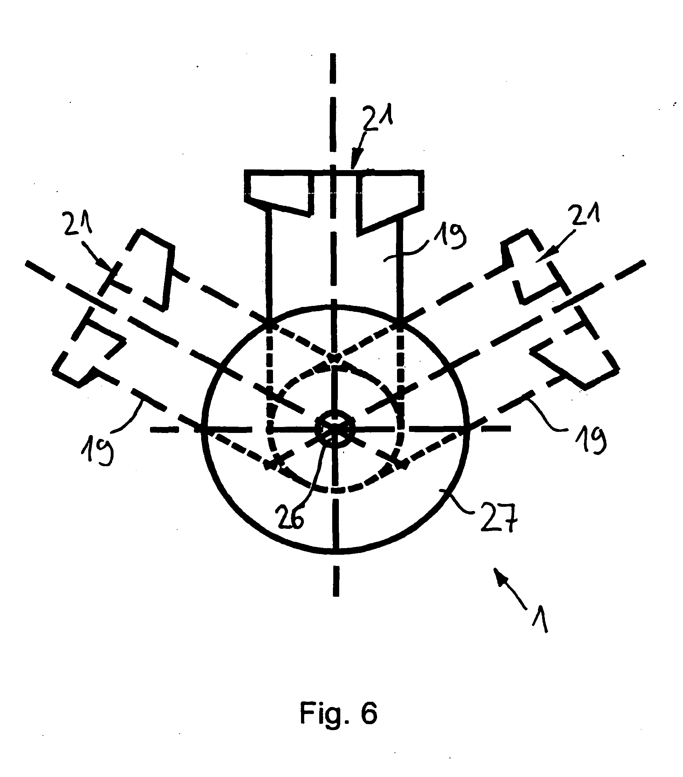

- Fig.6: eine Ansicht auf die der HF-Nadelelektrode abgewandte Rückseite des chirurgischen Instruments, wobei unterschiedliche Drehlagen eines verdrehbaren Gehäuseteils strichliniert angedeutet sind.

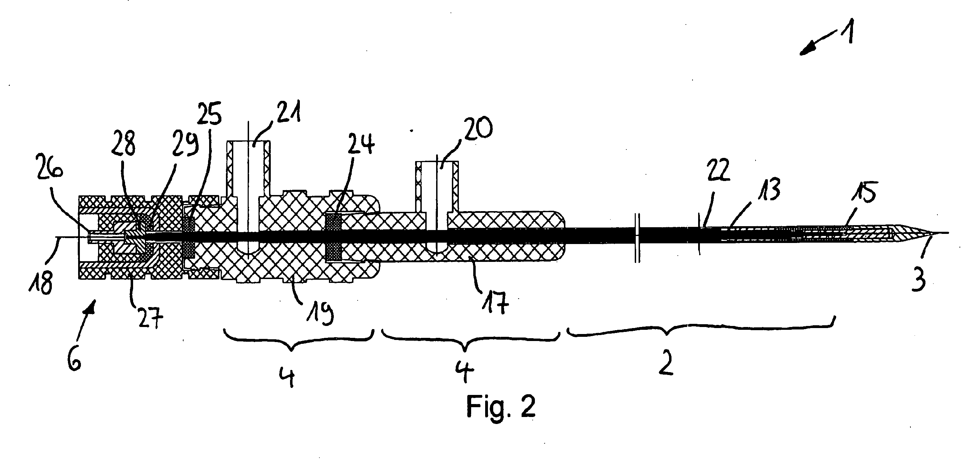

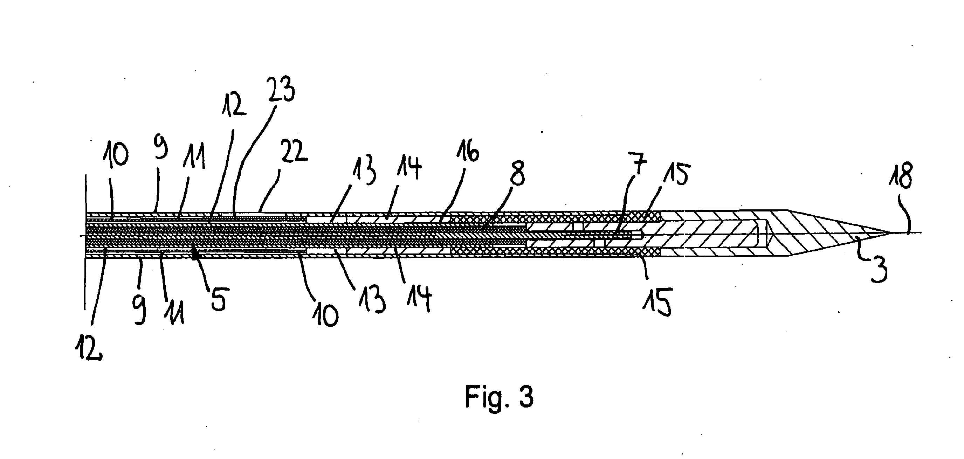

Ein im Ganzen mit 1 bezeichnetes chirurgisches Instrument zur Behandlung von tumorösem Gewebe hat einen Hohlschaft 2, der an seinem distalen Endbereich eine HF-Nadelelektrode 3 aufWeist und an seinem proximalen Endbereich mit einem Halteabschnitt 4 verbunden ist. Die HF-Nadelelektrode 3 ist über eine in dem Hohlschaft 2 axial verlaufende HF-Leitung 5 mit einem als HF-Anschluss dienenden Kupplungsteil 6 verbunden, an dem eine mit einem HF-Generator verbundene Zuleitung anschließbar ist. Die HF-Leitung hat einen elektrischen Leiter 7 und ein Dielektrikum 8, das den elektrischen Leiter 7 ummantelt. In Fig. 2 und 3 ist erkennbar, dass der Leiter 7 die HF-Nadelelektrode 3 elektrisch kontaktiert.

In Fig. 2 bis 5 ist erkennbar, dass der Hohlschaft 2 ein Außenrohr 9 und ein koaxial dazu angeordnetes Innenrohr 10 aufweist. In dem Innenrohr 10 ist die HF-Leitung 5 konzentrisch und mit radialem Abstand zum Innenrohr 10 angeordnet. Zwischen dem Außenrohr 9 und dem Innenrohr 10 ist ein erster Fluidkanalabschnitt 11 eines Fluidkanals gebildet. Ein zweiter Fluidkanalabschnitt 12 des Fluidkanals ist zwischen der HF-Leitung 5 und dem Innenrohr 10 gebildet.

Der erste Fluidkanalabschnitt 11 und der zweite Fluidkanalabschnitt 12 sind über eine Flüssigkeitskammer 13 miteinander verbunden und in Reihe geschaltet. Die Flüssigkeitskammer 1 3 ist benachbart zu der HF-Nadelelektrode 3 angeordnet und über ein Schweißstück 14 und eine elektrisch isolierende Hülse 15 wärmeleitend mit der HF-Nadelelektrode 3 verbunden. Das Schweißstück 14 grenzt mit einer Stirnwand an die Flüssigkeitskammer 13 an und liegt mit seiner Außenumfangswand flächig wärmeleitend an der Innenseite des Außenrohrs 9 an. Über die Hülse 15 ist die HF-Nadelelektrode 3 fest mit dem Außenrohr 9 verbunden.

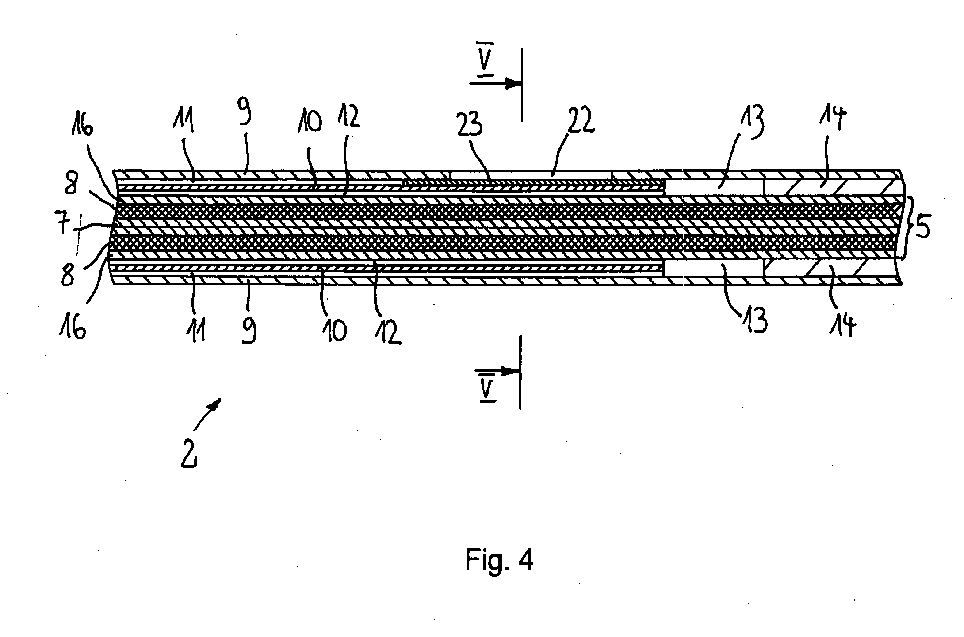

In Fig. 4 ist erkennbar, dass die Flüssigkeitskammer 13 die HF-Leitung 5 ringförmig umgrenzt. Dabei ist die Innenwand der Flüssigkeitskammer 13 durch eine Mantelschicht 16 der HF-Leitung 5 und die Außenwand der Flüssigkeitskammer 13 durch das Außenrohr 10 gebildet.

Der Halteabschnitt 4 hat ein erstes Gehäuseteil 17 und ein um die Längsmittelachse 18 des Hohlschafts 2 damit verdrehbar verbundenes zweites Gehäuseteil 19. An dem ersten Gehäuseteil 17 ist seitlich ein erster Fluidanschluss 20 und an dem zweiten Gehäuseteil 19 seitlich ein zweiter ersten Fluidanschluss 21 vorgesehen. Der erste Fluidanschluss 20 und der zweite Fluidanschluss 21 bilden jeweils einen Radialvorsprung, der etwa orthogonal zur Längsmittelachse 18 des Hohlschafts 2 angeordnet ist. Der erste Fluidanschluss 20 ist mit dem von der Flüssigkeitskammer 13 entfernten Ende des ersten Fluidkanalabschnitts 11 und der zweite Fluidanschluss 21 ist mit dem von der Flüssigkeitskammer 13 entfernten Ende des zweiten Fluidkanalabschnitts 12 verbunden. Somit ist in dem chirurgischen Instrument 1 ein Umlaufkanal gebildet, der sich von dem ersten Fluidanschluss 20 über den ersten Fluidkanalabschnitt 11, die Flüssigkeitskammer 13 und den zweiten Fluidkanalabschnitts 12 zu dem zweiten Fluidanschluss 21 erstreckt. Durch diesen Umlaufkanal kann eine Flüssigkeit, wie z.B. eine Kochsalzlösung hindurchgeleitet werden.

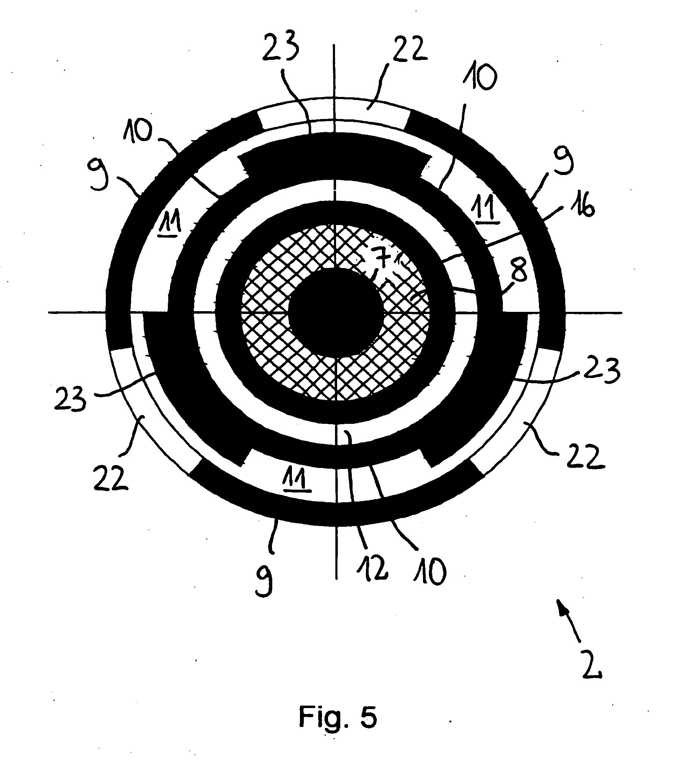

Wie in Fig. 5 erkennbar ist, weist das Außenrohr 9 an seinem distalen Endbereich mehrere mit dem Fluidkanal verbundene Austrittsöffnung 22 auf, die zur Abgabe der Flüssigkeit in das zu behandelnde tumoröse Gewebe dienen. Die Austrittsöffnungen 22 sind zwischen dem ersten Fluidanschluss 20 und der Flüssigkeitskammer 1 3 benachbart zu dieser angeordnet und in Umfangsrichtung des Außenrohrs 9 voneinander beabstandet.

An der Austrittsöffnung 22 ist ein als Schieber ausgebildetes Stellelement 23 angeordnet, mit dem der freie Durchlassquerschnitt der Austrittsöffnungen 22 variiert und die Austrittsöffnung 22 bei Bedarf auch ganz verschlossen oder ganz geöffnet werden kann. Das Stellelement 23 ist vorzugsweise durch eine Schweißstelle fest mit dem Innenrohr 10 verbunden und erstreckt sich abschnittweise in Umfangsrichtung einer zylindrischen Fläche, die zwischen dem Innenrohr 10 und dem Außenrohr 9 verläuft und zur Längsmittelachse 18 des Hohlschafts 2 konzentrisch ist. Durch Verdrehen des zweiten Gehäuseteils 19 und des damit verbundenen Innenrohrs 10 relativ zum ersten Gehäuseteil 17 und dem daran angeordneten Außenrohr 9 um die Längsmittelachse 18 (siehe Fig. 5) kann der Durchlassquerschnitt der Austrittsöffnungen 22 verändert werden. Die beiden Gehäuseteile 17, 18 dienen also auch als Betätigungselemente für das Stellelement 23. Das Innenrohr dient als Übertragungselement, über das die Stellelemente 23 mit dem zweiten Gehäuseteil 19 in Antriebsverbindung stehen. Das Außenrohr 9 ist an einem von dem ersten Gehäuseteil 17 entfernten Endbereich verdrehbar auf der mit dem Innenrohr 10 verbundenen Hülse 15 gelagert.

Erwähnt werden soll noch, dass zwischen dem ersten Gehäuseteil 17 und dem zweiten Gehäuseteil 19 eine die HF-Leitung 5 umgrenzende erste Dichtung 24 und zwischen dem zweiten Gehäuseteil 19 und dem Kupplungsteil 6 eine die HF-Leitung 5 umgrenzende zweite Dichtung 25 angeordnet ist. Das Kupplungsteil 6 hat ein mit dem Leiter 7 verbundenes Kontaktelement 26, das durch ein Distanzstück von einem das Kontaktelement 26 umgrenzenden dritten Gehäuseteil 27 beabstandet ist. Das dritte Gehäuseteil 27 ist auf das zweite Gehäuseteil 19 aufgesteckt. Axial ist das Kontaktelement 26 durch ein den Leiter 7 umgrenzendes Hüllelement 28 und eine Distanzscheibe 29 beabstandet.

## Patentansprüche

1. Chirurgisches Instrument (1) zur Behandlung von tumorösem Gewebe, mit einem Hohlschaft (2), der an seinem distalen Endbereich eine HF-Nadelelektrode (3) aufweist und an seinem proximalen Endbereich mit mindestens einem Halteabschnitt (4) verbunden ist, wobei in dem Hohlschaft (2) ein Fluidkanal vorgesehen ist, der sich im Wesentlichen in Axialrichtung des Hohlschafts (2) erstreckt, wobei der Fluidkanal einen ersten Fluidanschluss (20) und einen zweiten Fluidanschluss (21) aufweist, die von der HF-Nadelelektrode (3) beabstandet sind, wobei der Fluidkanal in dem Hohlschaft (2) einen ersten Fluidkanalabschnitt (11) und einen damit in Reihe geschalteten zweiten Fluidkanalabschnitt (12) hat, die in einem zwischen den Fluidanschlüssen (20, 21) und dem distalen Endbereich des Hohlschafts (2) und/oder in dem distalen Endbereich befindlichen Schaftabschnitt miteinander verbunden sind, wobei der Hohlschaft (2) ein Außerohr (9) und ein darin befindliches Innenrohr (10) aufweist, wobei der erste Fluidkanalabschnitt (11) zwischen dem Außenrohr (9) und dem Innenrohr (10) und der zweite Fluidkanalabschnitt (12) in dem Innenrohr (10) angeordnet sind, und wobei der Hohlschaft (2) zur Abgabe von Fluid in das tumoröse Gewebe an seinem distalen Endbereich mindestens eine mit dem Fluidkanal verbundene Austrittsöffnung (22) aufweist, **dadurch gekennzeichnet, dass** die HF-Nadelelektrode (3) mit einer den Hohlschaft (2) axial durchsetzenden HF-Leitung (5) verbunden ist, dass an der mindestens einen Austrittsöffnung (22) wenigstens ein Stellelement (23) zum Verändern des freien Durchlassquerschnitts der Austrittsöffnung (22) angeordnet ist, dass in dem Hohlschaft (2) ein mit dem Stellelement (23) in Betätigungsverbindung stehendes Übertragungselement vorgesehen ist, das sich im Wesentlichen in Axialrichtung des Hohlschafts (2) erstreckt und mit einem von dem Stellelement (23) beabstandeten Betätigungselement verbunden ist, dass das Stellelement (23) an dem Innenrohr (10) angeordnet oder durch einen Wandungsbereich des Innenrohrs (10) gebildet ist, und dass das Innenrohr (10) relativ zu dem Außenrohr (9) axial verschiebbar und/oder verdrehbar gelagert: ist und somit als Übertragungselement dient und dass das Innenrohr (10) sowie das Außerohr (9) jeweils an ihren proximalen Endbreichen mit einem das Betätigungselement bildenden Halteabschnitt (4) verbunden sind.

2. Chirurgisches Instrument (1) nach Anspruch 1, **dadurch gekennzeichnet, dass** benachbart zu der HF-Nadelelektrode (3) und/oder der mindestens einen Austrittsöffnung (22) zwischen dem ersten Fluidkanalabschnitt (11) und dem zweiten Huidkanalabschnitt (12) eine Flüssigkeitskammer (13) in dem Hohlschaft (2) vorgesehen ist, die wärmeleitend mit der HF-Nadelelektrode (3) und/oder einem Außenwandbereich des Hohlschafts (2) verbunden ist.

3. Chirurgisches Instrument (1) nach Anspruch 1 oder 2, **dadurch gekennzeichnet, dass** das Stellelement (23) als Schieber ausgebildet ist.

4. Chirurgisches Instrument (1) nach einem der Ansprüche 1 bis 3, **dadurch gekennzeichnet, dass** das Außenrohr (9) drehfest mit der HF-Nadelelektrode (3) verbunden ist.

5. Chirurgisches Instrument (1) nach einem der Ansprüche 1 bis 4, **dadurch gekennzeichnet, dass** der mindestens eine Fluidanschluss (20, 21) seitlich am proximalen Endbereich des chirurgischen Instruments (1) und ein mit der HF-Leitung (5) verbundener HF-Anschluss an dem von der HF-Nadelelektrode (3) beabstandeten Rückseite des chirurgischen Instruments (1) angeordnet sind.

## Claims

1. Surgical instrument (1) for treating tumorous tissue, with a hollow shaft (2) which, at its distal end area, has an HF needle electrode (3) and, at its proximal end area, is connected to at least one holding section (4), the hollow shaft (2) accommodating a fluid channel that extends substantially in the axial direction of the hollow shaft (2), the fluid channel having a first fluid attachment (20) and a second fluid attachment (21) which are at a distance from the HF needle electrode (3), the fluid channel in the hollow shaft (2) having a first fluid channel section (11) and, coupled in series with the latter, a second fluid channel section (12) that are connected to each other in a shaft section located between the fluid attachments (20, 21) and the distal end area of the hollow shaft (2) and/or in the distal end area, the hollow shaft (2) having an outer tube (9) and, located in the latter, an inner tube (10), the first fluid channel section (11) being arranged between the outer tube (9) and the inner tube (10), and the second fluid channel section (12) being arranged in the inner tube (10), and the distal end area of the hollow shaft (2) having, for the purpose of delivering fluid into the tumorous tissue, at least one outlet opening (22) connected to the fluid channel, **characterized in that** the HF needle electrode (3) is connected to an HF wire (5) passing axially through the hollow shaft (2), **in that** at least one adjustment element (23) for changing the free cross section of passage through the outlet opening (22) is arranged at the at least one outlet opening (22), **in that** the hollow shaft (2) accommodates a transmission element which is operatively connected to the adjustment element (23) and which extends substantially in the axial direction of the hollow shaft (2) and is connected to an operating element at a distance from the adjustment element (23), **in that** the adjustment element (23) is arranged on the inner tube (10) or is formed by a wall area of the inner tube (10), **in that** the inner tube (10) is mounted in such a way as to be axially displaceable and/or rotatable relative to the outer tube (9) and thus serves as transmission element, and **in that** the inner tube (10) and the outer tube (9) are each connected at their proximal end areas to a holding section (4) that forms the operating element.

2. Surgical instrument (1) according to Claim 1,
**characterized in that**, adjacent to the HF needle electrode (3) and/or to the at least one outlet opening (22), a liquid chamber (13) is present in the hollow shaft (2) between the first fluid channel section (11) and the second fluid channel section (12), which liquid chamber (13) is connected heat-conductively to the HF needle electrode (3) and/or to an outer wall area of the hollow shaft (2).

3. Surgical instrument (1) according to Claim 1 or 2, **characterized in that** the adjustment element (23) is designed as a slide.

4. Surgical instrument (1) according to one of Claims 1 to 3, **characterized in that** the outer tube (9) is connected to the HF needle electrode (3) in a rotationally fixed manner.

5. Surgical instrument (1) according to one of Claims 1 to 4, **characterized in that** the at least one fluid attachment (20, 21) is arranged laterally on the proximal end area of the surgical instrument (1), and an HF attachment connected to the HF wire (5) is arranged on the rear face of the surgical instrument (1) at a distance from the HF needle electrode (3).

## Revendications

1. Instrument chirurgical (1) destiné au traitement de tissus tumoraux et présentant :
une tige creuse (2) dotée d'une électrode HF à aiguille (3) sur sa partie d'extrémité distale et reliée par sa partie d'extrémité proximale à au moins un transport de matière (4),
un canal pour fluide qui s'étend essentiellement dans la direction axiale de la tige creuse (2) étant prévu dans la tige creuse (2),
le canal pour fluide présentant un premier raccordement pour fluide (20) et un deuxièmement raccordement pour fluide (21) situés à distance de l'électrode HF à aiguille (3),
le canal pour fluide présentant dans la tige creuse (2) un premier tronçon (11) de canal pour fluide et un deuxième tronçon (12) de canal pour fluide raccordé en série au premier et reliés l'un à l'autre dans un tronçon de tige situé entre les raccordements pour fluide (20, 21) et l'extrémité distale de la tige creuse (2) et/ou dans l'extrémité distale,
la tige creuse (2) présentant un tube extérieur (9) dans lequel est situé un tube intérieur (10), le premier tronçon (11) de canal pour fluide étant disposé entre le tube extérieur (9) et le tube intérieur (10) et le deuxième tronçon (12) de canal pour fluide étant disposé dans le tube intérieur (10),
la tige creuse (2) présentant au moins une ouverture de sortie (22) reliée au canal pour fluide pour délivrer un fluide dans le tissu tumoral par sa partie d'extrémité distale,
**caractérisé en ce que**
l'électrode HF à aiguille (3) est reliée à un conducteur HF (5) qui traverse axialement la tige creuse (2),
**en ce qu'**au moins un élément d'ajustement (23) qui modifie la section transversale de passage libre de l'ouverture de sortie (22) est disposé sur la ou les ouvertures de sortie (22),
**en ce qu'**un élément de transfert placé en liaison d'actionnement avec l'élément d'ajustement (23), qui s'étend essentiellement dans la direction axiale de la tige creuse (2) et qui est relié à un élément d'actionnement situé à distance de l'élément d'ajustement (23), est prévu dans la tige creuse (2),
**en ce que** l'élément d'ajustement (23) est disposé sur le tube intérieur (10) ou est formé par une partie de la paroi du tube intérieur (10),
**en ce que** le tube intérieur (10) est monté de manière à pouvoir coulisser axialement et/ou à pouvoir tourner par rapport au tube extérieur (9) et sert ainsi d'élément de transfert et
**en ce que** le tube intérieur (10) ainsi que le tube extérieur (9) sont reliés par leur partie d'extrémité proximale à un tronçon de maintien (4) qui forme l'élément d'actionnement.

2. Instrument chirurgical (1) selon la revendication 1, **caractérisé en ce qu'**une chambre à liquide (13) qui est reliée de manière thermiquement conductrice à l'électrode HF à aiguille (3) et/ou à une partie de paroi extérieure de la tige creuse (2) est prévue dans la tige creuse (2) et entre le premier tronçon (11) de canal pour fluide et le deuxième tronçon (12) de canal pour fluide au voisinage de l'électrode HF à aiguille (3) et/ou de la ou des ouvertures de sortie (22).

3. Instrument chirurgical (1) selon les revendications 1 ou 2, **caractérisé en ce que** l'élément de réglage (23) est configuré comme coulisseau.

4. Instrument chirurgical (1) selon l'une des revendications 1 à 3, **caractérisé en ce que** le tube extérieur (9) est relié à rotation solidaire à l'électrode HF à aiguille (3).

5. Instrument chirurgical (1) selon l'une des revendications 1 à 4, **caractérisé en ce que** le ou les raccordements pour fluide (20, 21) sont disposés latéralement sur la partie d'extrémité proximale de l'instrument chirurgical (1) et **en ce qu'**un raccordement HF relié au conducteur HF (5) est disposé sur le côté arrière, situé à distance de l'électrode HF à aiguille (3), de l'instrument chirurgical (1).
